# EUROPEAN PATENT APPLICATION

(11) **EP 4 534 530 A1**
(43) Date of publication of application: **09.04.2025**
(21) Application number: 23815198.9
(22) Date of filing: 30.05.2023
(51) Int. Cl.: C07D 221/04, C07D 215/26, C07D 215/28, A61K 31/47, A61P 31/00, A61P 35/00

(54) **7-CYANO-8-HYDROXYQUINOLINE DERIVATIVE, PREPARATION METHOD THEREFOR AND MEDICAL USE THEREOF**

(30) Priority: 31.05.2022 CN 202210610140
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: DENG, Yijun, Shanghai 201203 (CN); WU, Liang, Shanghai 201203 (CN); ZHU, Haitao, Shanghai 201203 (CN); ZHOU, Chen, Shanghai 201203 (CN)
(74) Representative: Regimbeau
(86) International application number: PCT/CN2023/097084
(87) International publication number: WO 2023/232019

(57) **Abstract**

Provided are a 7-cyano-8-hydroxyquinoline derivative, a preparation method therefor and a medical use thereof. Specifically, disclosed are a compound represented by general formula (I), a preparation method therefor, a pharmaceutical composition containing same, and a use thereof in the treatment of infectious diseases or cancer. The compound has excellent antibacterial, antiviral and antitumor activity, and can be developed as a drug for the treatment of infectious diseases or tumors. The definitions of groups in the general formula (I) are the same as in the description.

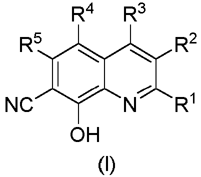

## Description

### FIELD OF THE INVENTION

The present invention relates to 7-cyano-8-hydroxyquinoline derivative, preparation method therefor and medical use thereof.

### BACKGROUND OF THE INVENTION

Nitroxoline, the chemical name of which is 5-nitro-8-hydroxyquinoline, was developed as an oral antibiotic drug in the 1960s. It was mainly used for urinary tract infections and had a relatively safe history of use. Also, latest studies have found that nitroxoline can exert a synergistic inhibitory effect on tumor angiogenesis, as well as an inhibitory effect on the proliferation of tumor cells. However, the activity of the current quinoline derivatives still needs further improvement. On this basis, the applicant has studied 7-cyano-8-hydroxyquinoline derivatives.

### SUMMARY OF THE INVENTION

The inventor has conducted painstaking research to design and synthesize a series of 7-cyano-8-hydroxyquinoline derivatives, which show excellent antibacterial or antitumor activity and can be developed as drugs for treating infectious diseases or tumors.

The present invention provides a compound of formula (I), a stereoisomer thereof, or a pharmaceutically acceptable salt thereof: 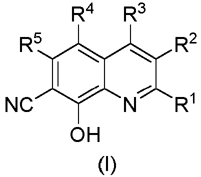 wherein:
R¹ is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R² is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R³ is selected from the group consisting of hydrogen atom, alkyl, halogen, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R⁴ is selected from the group consisting of hydrogen atom, halogen, haloalkyl, carboxy, hydroxy, hydroxyalkyl, alkoxy, haloalkoxy, amino, thiol, alkenyl, alkynyl, cycloalkyl, heterocyclyl, aryl and heteroaryl;
R⁵ is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
provided that R¹ to R⁵ are not all hydrogen atoms at the same time.

In some embodiments, in the compound of formula (I), R⁴ is selected from the group consisting of hydrogen atom, halogen, haloC₁₋₆ alkyl, carboxy and hydroxy, preferably selected from the group consisting of hydrogen atom, halogen, haloC₁₋₆ alkyl and carboxy, more preferably selected from the group consisting of hydrogen atom, bromine atom, chlorine atom, trifluoromethyl and carboxy.

In some other embodiments, in the compound of formula (I), R³ is selected from the group consisting of hydrogen atom, C₁₋₆ alkyl, halogen and hydroxy, preferably hydrogen atom or C₁₋₆ alkyl, more preferably hydrogen atom or methyl.

In some other embodiments, in the compound of formula (I), R¹ is selected from the group consisting of hydrogen atom, halogen, C₁₋₆ alkyl and hydroxy, preferably hydrogen atom or halogen, more preferably hydrogen atom or fluorine atom.

In some other embodiments, in the compound of formula (I), R⁵ is selected from the group consisting of hydrogen atom, halogen, alkyl and hydroxy, preferably hydrogen atom or halogen, more preferably hydrogen atom or chlorine atom.

In some other embodiments, in the compound of formula (I), R² is selected from the group consisting of hydrogen atom, halogen, C₁₋₆ alkyl and hydroxy, preferably hydrogen atom.

In some other embodiments, the compound of formula (I) is a compound of formula (II): 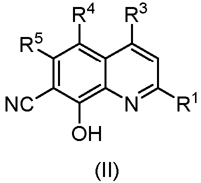 wherein:
R¹ is hydrogen atom or halogen, preferably hydrogen atom or fluorine atom;
R³ is hydrogen atom or C₁₋₆ alkyl, preferably hydrogen atom or methyl;
R⁴ is selected from the group consisting of hydrogen atom, halogen, haloC₁₋₆ alkyl and carboxy, preferably selected from the group consisting of hydrogen atom, bromine atom, chlorine atom, trifluoromethyl and carboxy;
R⁵ is hydrogen atom or halogen, preferably hydrogen atom or chlorine atom;
provided that R¹ to R⁵ are not all hydrogen atoms at the same time.

Typical compounds of formula (I) of the present invention include, but are not limited to the following compounds:

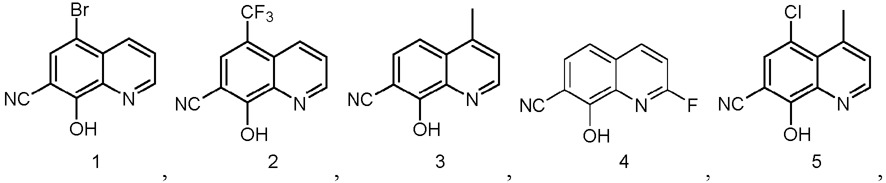

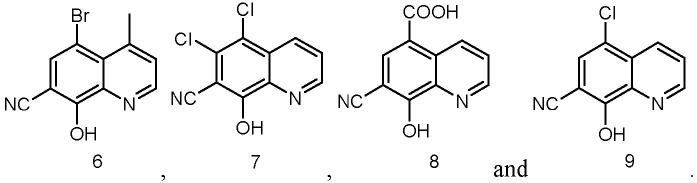

The present invention also provides a method for preparing a compound of formula (I), a stereoisomer thereof, or a pharmaceutically acceptable salt thereof, comprising the following steps of:
method I: 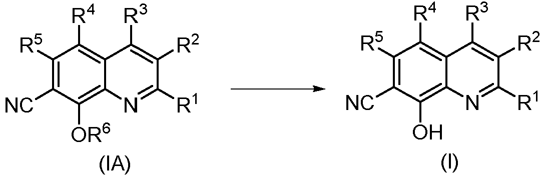
subjecting a compound of formula (IA) to a dealkylation reaction in the presence of lithium chloride to form the compound of formula (I), wherein R⁶ is alkyl, preferably C₁₋₆ alkyl, more preferably methyl;
further, 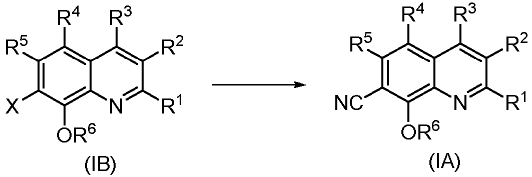
reacting a compound of formula (IA) with zinc cyanide to form the compound of formula (IA), wherein X is a halogen, preferably bromine atom or chlorine atom;
method II: 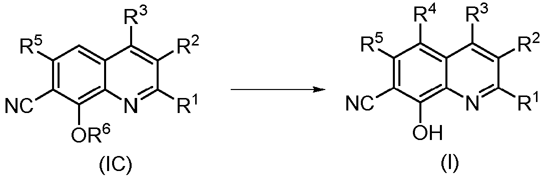
reacting a compound of formula (IA) with a halogenating reagent to form the compound of formula (IA), wherein the halogenating reagent is selected from the group consisting of N-chlorosuccinimide, N-bromosuccinimide, N-iodosuccinimide, pyridinium tribromide, cuprous chloride and cuprous iodide, preferably N-chlorosuccinimide or N-bromosuccinimide, R⁴ is halogen, preferably chlorine atom or bromine atom;
R¹ to R³ and R⁵ are as defined in formula (I).

The present invention also provides a pharmaceutical composition, comprising a therapeutically amount of the compound of formula (I), the stereoisomer thereof, or the pharmaceutically acceptable salt thereof, and one or more pharmaceutically acceptable excipients.

The present invention relates to use of the compound of formula (I'), the stereoisomer thereof, or the pharmaceutically acceptable salt thereof, or a pharmaceutical composition comprising the same, in the preparation of a medicament for treating infectious disease or cancer, 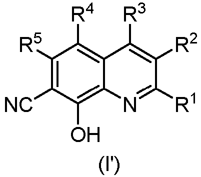 wherein, R¹ to R³ and R⁵ are as defined in formula (I);
R⁴ is selected from the group consisting of hydrogen atom, halogen, alkyl, haloalkyl, carboxy, hydroxy, hydroxyalkyl, alkoxy, haloalkoxy, amino, thiol, alkenyl, alkynyl, cycloalkyl, heterocyclyl, aryl and heteroaryl; preferably, R⁴ is selected from the group consisting of hydrogen atom, halogen, C₁₋₆ alkyl, haloC₁₋₆ alkyl, carboxy and hydroxy;
in particular, the compound of formula (I') includes, but is not limited to the following compounds: 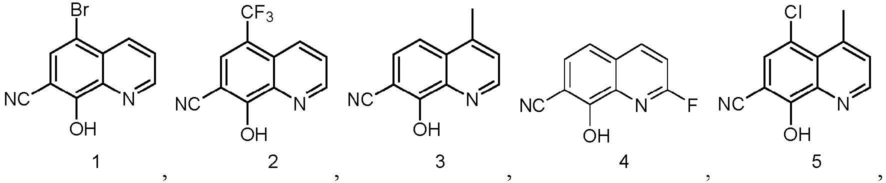 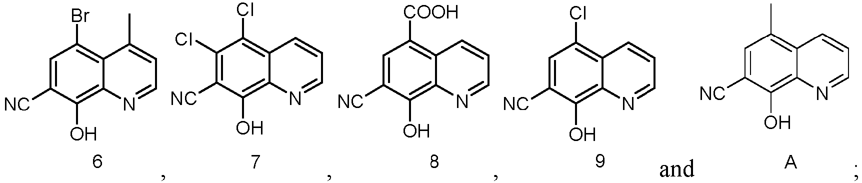
wherein, the infectious disease is preferably systemic infection, reproductive system infection or urinary tract infection; particularly, the infectious disease is caused by a gram-negative bacterium, a gram-positive bacterium, a virus or a fungus; more particularly, the gram-negative bacterium includes *Escherichia coli, Acinetobacter baumannii* and *Klebsiella pneumoniae,* the *Escherichia coli* is preferably a carbapenem-resistant *Escherichia coli,* and the *Acinetobacter baumannii* is preferably a carbapenem-resistant *Acinetobacter baumannii;* the gram-positive bacterium includes *Staphylococcus aureus,* the *Staphylococcus aureus* is preferably a methicillin-resistant and/or -susceptible *Staphylococcus aureus;* the virus includes human papillomavirus, preferably one or more of *Human Papillomavirus 6 (HPV6), Human Papillomavirus 11 (HPV11), Human Papillomavirus 16 (HPV16)* and *Human Papillomavirus 18 (HPV18);* more preferably *Human papillomavirus 6, Human Papillomavirus 11, Human papillomavirus 16* or *Human Papillomavirus 18;*
wherein, the cancer is preferably bladder cancer or prostate cancer.

The pharmaceutical composition of the present invention can be various conventional dosage forms, for example, tablet, aqueous suspension, oil suspension, dispersible powder, dispersible granule, emulsion, hard capsule, soft capsule, sterile aqueous solution for injection, sterile oil-in-water microemulsion for injection, or suppository. Each of the above dosage forms can be prepared by conventional preparation methods.

### Definition of Terms

The terms not defined herein have the meanings commonly understood by those skilled in the art. The terms defined herein have the meanings set forth in the description.

The term "substitution" or "substituent group" refers to the substitution of one or more hydrogen atoms by indicated group(s). The substitution can be at any position when the substitution position is not specified, but only if a stable or chemically feasible chemical is formed.

The term "optional" or "optionally" means that the event or circumstance described subsequently can, but need not occur, and such a description includes the situation in which the event or circumstance occurs, and the situation in which the event or circumstance does not occur.

When any variable, for example R, appears more than once in the structure of a compound, its definition in each case is independent. For example, if a group is substituted by 0-2 R, the group can optionally be substituted by up to 2 R, and there are independent options for R in each case.

The term "alkyl" refers to a saturated, linear or branched monovalent hydrocarbon group having 1-20 (for example, 1, 2, 3, 4, 5, 6, 7, 8, 9, 10, 11, 12, 13, 14, 15, 16, 17, 18, 19, and 20) carbon atoms, preferably C₁₋₁₀ alkyl, more preferably C₁₋₆ alkyl. Examples of alkyl include, but are not limited to, methyl, ethyl, n-propyl, isopropyl, n-butyl, isobutyl, *tert-butyl,* sec-butyl, n-pentyl, 2,2-dimethylpropyl, 2-methylbutyl, n-hexyl, 2,2-dimethylbutyl, 2-methylpentyl, 3-methylpentyl, 2,3-dimethylbutyl, n-heptyl, 2-methylhexyl, 3-methylhexyl, 2,3-dimethylpentyl, 2,4-dimethylpentyl, 2,2-dimethylpentyl, 3,3-dimethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 3-ethylhexyl, 4-ethylhexyl, 2-methyl-3-ethylpentyl, n-nonyl, 2-methyl-3-ethylhexyl, n-decyl, and 3,3-diethylhexyl.

The term "alkenyl" refers to a linear or branched monovalent hydrocarbon group having 2-6 (for example, 2, 3, 4, 5, and 6) carbon atoms and at least one carbon-carbon double bond, wherein the carbon-carbon double bond can be located anywhere within the alkenyl, preferably C₂₋₅ alkenyl. Examples of alkenyl include, but are not limited to, -CH=CH₂, -CH=CH-CH₃, -CH₂-CH=CH₂, -CH=CH-CH₂-CH₃, -CH₂-CH=CH-CH₃, -CH=CH-CH=CH₂, -CH=C(CH₃)-CH₃ and -CH₂-C(CH₃)=CH₂.

The term "alkynyl" refers to a linear or branched monovalent hydrocarbon group having 2-6 (for example, 2, 3, 4, 5, and 6) carbon atoms and at least one carbon-carbon triple bond, wherein the carbon-carbon triple bond can be located anywhere within the alkynyl, preferably C₂₋₅ alkynyl. Examples of alkynyl include, but are not limited to, -C≡CH, -C≡C-CH₃, -CH₂-C≡CH, -C≡C-CH₂-CH₃, -CH₂-CH₂-C≡CH, -CH(CH₃)C≡CH and -CH₂-C≡C-CH₃.

The term "cycloalkyl" includes two categories, one is conventional cycloalkyl and the other is heterostructured cycloalkyl.

The conventional cycloalkyl refers to an aliphatic, saturated or partially unsaturated monovalent cyclic hydrocarbon group having 3-20 (for example, 3, 4, 5, 6, 7, 8, 9, 10, 11, 12, 13, 14, 15, 16, 17, 18, 19, and 20) carbon atoms, preferably C₃₋₁₂ conventional cycloalkyl, more preferably C₃₋₁₀ conventional cycloalkyl, further preferably C₃₋₈ conventional cycloalkyl, most preferably C₃₋₆ conventional cycloalkyl. The conventional cycloalkyl optionally comprises one or more double or triple bonds.

The conventional cycloalkyl can be a monocyclic cycloalkyl, and examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl, and cyclooctyl. The conventional cycloalkyl can also be a polycyclic cycloalkyl (for example, dicycloalkyl and tricycloalkyl), and polycyclic cycloalkyl includes spiro cycloalkyl, fused cycloalkyl and bridged cycloalkyl.

The term "spiro cycloalkyl" refers to a 5-20 membered (for example, 5, 6, 7, 8, 9, 10, 11, 12, 13, 14, 15, 16, 17, 18, 19, and 20 membered) spiro cycloalkyl, preferably 6-14 membered spiro cycloalkyl, more preferably 7-10 membered spiro cycloalkyl. The spiro cycloalkyl can be a mono-spiro cycloalkyl, di-spiro cycloalkyl or poly-spiro cycloalkyl, preferably mono-spiro cycloalkyl, preferably 4-membered/4-membered, 4-membered/5-membered, 4-membered/6-membered, 5-membered/5-membered or 5-membered/6-membered mono-spiro cycloalkyl. Examples of spiro cycloalkyl include, but are not limited to:

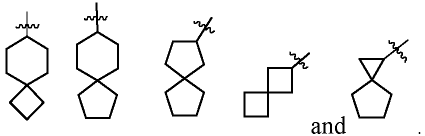

The term "fused cycloalkyl" refers to a 5-20 membered (for example, 5, 6, 7, 8, 9, 10, 11, 12, 13, 14, 15, 16, 17, 18, 19, and 20 membered) fused cycloalkyl, preferably 6-14 membered fused cycloalkyl, more preferably 7-10 membered fused cycloalkyl. The fused cycloalkyl can be a bicyclic, tricyclic, tetracyclic or pentacyclic or more fused cycloalkyl, preferably bicyclic or tricyclic fused cycloalkyl, more preferably 5-membered/5-membered or 5-membered/6-membered fused cycloalkyl. Examples of fused cycloalkyl include, but are not limited to:

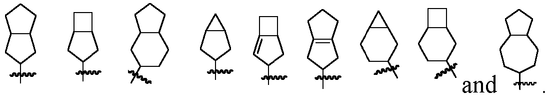

The term "bridged cycloalkyl" refers to a 5-20 membered (for example, 5, 6, 7, 8, 9, 10, 11, 12, 13, 14, 15, 16, 17, 18, 19, and 20) bridged cycloalkyl, preferably 6-14 membered bridged cycloalkyl, more preferably 7-10 membered bridged cycloalkyl. The bridged cycloalkyl can be a bicyclic, tricyclic, tetracyclic or pentacyclic or more bridged cycloalkyl, preferably bicyclic, tricyclic or tetracyclic bridged cycloalkyl, more preferably bicyclic or tricyclic bridged cycloalkyl. Examples of bridged cycloalkyl include, but are not limited to:

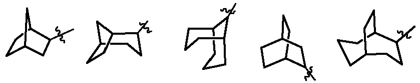

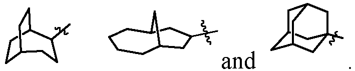

The term "heterostructured cycloalkyl" includes monocyclic cycloalkyl, spiro cycloalkyl, fused cycloalkyl, and bridged cycloalkyl that are fused with any one selected from the group consisting of conventional aryl, conventional heteroaryl, and conventional heterocyclyl, with the attachment point located on the corresponding conventional cycloalkyl (i.e., on the monocyclic cycloalkyl, spiro cycloalkyl, fused cycloalkyl, or bridged cycloalkyl). Examples of heterostructured cycloalkyl include, but are not limited to:

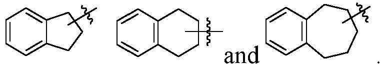

The term "heterocyclyl" includes two categories, one is conventional heterocyclyl and the other is heterostructured heterocyclyl.

The conventional heterocyclyl refers to an aliphatic, saturated or partially unsaturated monovalent cyclic hydrocarbon group having 3-20 (for example, 3, 4, 5, 6, 7, 8, 9, 10, 11, 12, 13, 14, 15, 16, 17, 18, 19, and 20) ring atoms, wherein one or more ring atoms are replaced by one or more elements selected from the group consisting of nitrogen, oxygen, S, S(O), and S(O)₂, and the replacement does not form -O-O-, - O-S-or -S-S-; preferably C₃₋₁₂ conventional heterocyclyl, wherein 1-4 (for example, 1, 2, 3, and 4) ring atoms are heteroatoms; more preferably C₃₋₈ conventional heterocyclyl, wherein 1-3 (for example, 1, 2, and 3) ring atoms are heteroatoms; most preferably C₅₋₇ conventional heterocyclyl, wherein 1-2 or 1-3 ring atoms are heteroatoms.

The conventional heterocyclyl can be a monocyclic heterocyclyl, and examples of monocyclic heterocyclyl include, but are not limited to, oxetanyl, 3-pyrrolinyl, pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and pyranyl, preferably 1,2,5-oxadiazolyl, pyranyl or morpholinyl. The conventional heterocyclyl can also be a polycyclic heterocyclyl, and polycyclic heterocyclyl includes spiro heterocyclyl, fused heterocyclyl and bridged heterocyclyl.

The term "spiro heterocyclyl" refers to a 5-20 membered (for example, 5, 6, 7, 8, 9, 10, 11, 12, 13, 14, 15, 16, 17, 18, 19, and 20 membered) spiro heterocyclyl, preferably 6-14 membered spiro heterocyclyl, more preferably 7-10 membered spiro heterocyclyl. The spiro heterocyclyl can be a mono-spiro heterocyclyl, di-spiro heterocyclyl or poly-spiro heterocyclyl, preferably mono-spiro heterocyclyl or di-spiro heterocyclyl, more preferably 3-membered/6-membered, 4-membered/4-membered, 4-membered/5-membered, 4-membered/6-membered, 5-membered/5-membered or 5-membered/6-membered mono-spiro heterocyclyl. Examples of spiro heterocyclyl include, but are not limited to:

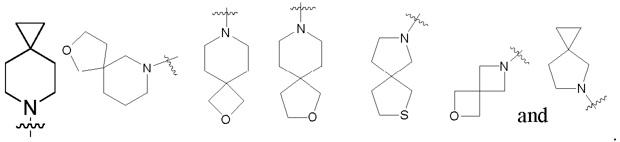

The term "fused heterocyclyl" refers to a 5-20 membered (for example, 5, 6, 7, 8, 9, 10, 11, 12, 13, 14, 15, 16, 17, 18, 19, and 20 membered) fused heterocyclyl, preferably 6-14 membered fused heterocyclyl, more preferably 7-10 membered fused heterocyclyl. The fused heterocyclyl can a bicyclic, tricyclic, tetracyclic or pentacyclic or more fused heterocyclyl, preferably bicyclic or tricyclic fused heterocyclyl, more preferably 5-membered/5-membered or 5-membered/6-membered bicyclic fused heterocyclyl. Examples of fused heterocyclyl include, but are not limited to:

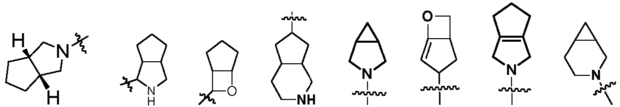

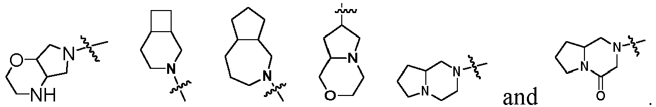

The term "bridged heterocyclyl" refers to a 5-14 membered (for example, 5, 6, 7, 8, 9, 10, 11, 12, 13, and 14 membered) bridged heterocyclyl, preferably 6-14 membered bridged heterocyclyl, more preferably 7-10 membered bridged heterocyclyl. The bridged heterocyclyl can be a bicyclic, tricyclic, tetracyclic or pentacyclic or more bridged heterocyclyl, preferably bicyclic, tricyclic or tetracyclic bridged heterocyclyl, more preferably bicyclic or tricyclic bridged heterocyclyl. Examples of bridged heterocyclyl include, but are not limited to:

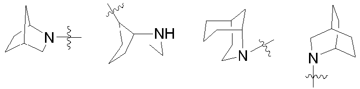

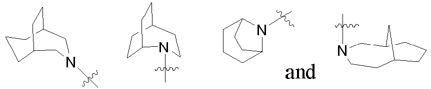

The term "heterostructured heterocyclyl" includes monocyclic heterocyclyl, spiro heterocyclyl, fused heterocyclyl, and bridged heterocyclyl that are fused with any one selected from the group consisting of conventional aryl, conventional heteroaryl, and conventional cycloalkyl, with the attachment point located on the corresponding conventional heterocyclyl (i.e., on the monocyclic heterocyclyl, spiro heterocyclyl, fused heterocyclyl or bridged heterocyclyl). Examples of heterostructured heterocyclyl include, but are not limited to:

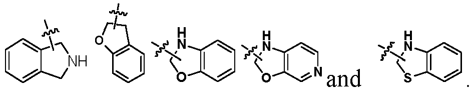

The term "aryl" includes two categories, one is conventional aryl and the other is heterostructured aryl.

Conventional aryl refers to a 6-14 membered (for example, 6, 7, 8, 9, 10, 11, 12, 13, and 14 membered) aromatic hydrocarbon group, preferably C₆₋₁₀ conventional aryl, more preferably phenyl, naphthyl, phenanthryl or anthracenyl.

The term "heterostructured aryl" includes a conventional aryl that is fused with any one selected from the group consisting of conventional heteroaryl, conventional heterocyclyl, and conventional cycloalkyl, with the attachment point located on the conventional aryl. Examples of heterostructured aryl include, but are not limited to:

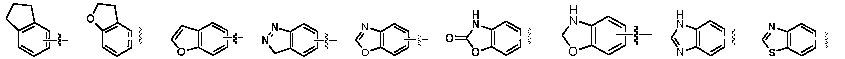

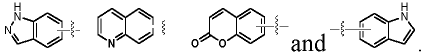

The term "heteroaryl" includes two categories, one is conventional heteroaryl and the other is heterostructured heteroaryl.

The conventional heteroaryl refers to a 5-14 membered (for example, 5, 6, 7, 8, 9, 10, 11, 12, 13, and 14 membered) aromatic hydrocarbon group, wherein 1-4 (for example, 1, 2, 3 and 4) carbon atoms are replaced by heteroatoms, and the heteroatoms are selected from the group consisting of oxygen, sulfur, and nitrogen. Preferably, the number of ring atoms is 5-10, including 1-3 (for example, 1, 2, and 3) heteroatoms. More preferably, the number of ring atoms is 5 or 6, including 1-2 heteroatoms. Examples of conventional heteroaryl include, but are not limited to, imidazolyl, furyl, thienyl, thiazolyl, pyrazolyl, oxazolyl, pyrrolyl, tetrazolyl, pyridyl, pyrimidinyl, thiadiazolyl, and pyrazinyl, preferably imidazolyl, thiazolyl, pyrazolyl, pyrimidinyl or thiazolyl, more preferably pyrazolyl or thiazolyl.

The term "heterostructured heteroaryl" includes a conventional heteroaryl that is fused with any one selected from the group consisting of conventional aryl, conventional cycloalkyl, and conventional heterocyclyl, with the attachment point located on the conventional heteroaryl. Examples of heterostructured heteroaryl include, but are not limited to:

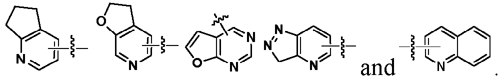

The term "alkoxy" includes -O-alkyl and -O-cycloalkyl, wherein "alkyl" and "cycloalkyl" are as defined above. Examples of alkoxy include, but are not limited to, methoxy, ethoxy, propoxy, butoxy, cyclopropyloxy, cyclobutyloxy, cyclopentyloxy, and cyclohexyloxy.

The term "haloalkyl" refers to an alkyl substituted by one or more halogens, wherein the alkyl is as defined above.

The term "haloalkoxy" refers to an alkoxy substituted by one or more halogens, wherein the alkoxy is as defined above.

The term "hydroxy" refers to -OH.

The term "halogen" refers to -F, -Cl, -Br or -I.

The term "amino" refers to -NH₂.

The term "cyano" refers to -CN.

The term "nitro" refers to -NO₂.

The term "oxo" refers to =O.

The term "carboxy" refers to -C(=O)OH.

The term "thiol" refers to -SH.

The term "alkoxycarbonyl" refers to -C(=O)O-alkyl or -C(=O)O-cycloalkyl, wherein, the alkyl and the cycloalkyl are as defined above.

The term "acyl" refers to -C(=O)R, wherein R is selected from the group consisting of alkyl, cycloalkyl, heterocyclyl, aryl and heteroaryl.

The symbol 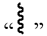 refers to attachment point.

The term "stereoisomer" refers to an isomer produced by the different spatial arrangement of atoms in a molecule, which can be classified into two types: cis-trans isomer and enantiomer, and can also be classified into two categories: enantiomer and diastereoisomer. A stereoisomer caused by rotation of a single bond is called a conformational isomer and sometimes a rotamer. A stereoisomer caused by bond length, bond angle, double bonds and rings within molecule, etc., is called a configurational isomer, and the configurational isomer is classified into two categories. Among them, an isomer caused by inability of a double bond or a single bond of ring-forming carbon atoms to rotate freely is called a geometric isomer, also known as cis-trans isomer, which is classified into two configurations, Z and E. For example, cis-2-butene and trans-2-butene are a pair of geometric isomers, and stereoisomers with different optical rotation properties caused by the absence of inverse axial symmetry in the molecule are called optical isomers, which are classified into R and S configurations. The "stereoisomer" described in the present invention may be understood to include one or more of the enantiomer, configurational isomer and conformational isomer described above, if not otherwise specified.

The term "pharmaceutically acceptable" means when used in the preparation of a pharmaceutical composition, the pharmaceutical composition is generally safe and non-toxic, meets the biological needs, and can be accepted and used as a drug for a mammal (for example, a human).

The term "pharmaceutically acceptable salt" should be understood as referring to the following salts, which are pharmaceutically acceptable salts and which possess the expected pharmacological activity of the parent compound (i.e., the compound of the formula). Such salts include:
(1) acid addition salts formed with inorganic acids, or acid addition salts formed with organic acids; wherein, the inorganic acids can be one or more of hydrochloric acid, hydrobromic acid, sulfuric acid, nitric acid and phosphoric acid; the organic acids can be one or more of formic acid, oxalic acid, succinic acid, acetic acid, benzenesulfonic acid, benzoic acid, camphorsulfonic acid, citric acid, ethanesulfonic acid, fumaric acid, glucoheptonic acid, gluconic acid, glutamic acid, glycollic acid, hydroxynaphthoic acid, 2-hydroxyethanesulfonic acid, lactic acid, maleic acid, malic acid, mandelic acid, methanesulfonic acid, muconic acid, 2-naphthalenesulfonic acid, propionic acid, salicylic acid, succinic acid, dibenzoyl-L-tartaric acid, tartaric acid, p-toluenesulfonic acid, trimethylacetic acid and trifluoroacetic acid; and
(2) salts formed when acid protons present in the parent compound are substituted by metal ions, for example, alkali metal ions (e.g., Na⁺, K⁺ or Li⁺), alkaline earth metal ions (such as Ca²⁺ or Mg²⁺) or aluminum ions; or, when coordinated with organic or inorganic bases; wherein, the organic bases can be one or more of pyridines, imidazoles, pyrazines, indoles, purines, tertiary amines and anilines, preferably one or more of pyridine, methylpyridine, 4-dimethylaminopyridine, 2-methyl-5-ethylpyridine, triethylamine, N,N-diisopropylethanamine, N,N-dimethylaniline, diethanolamine, ethanolamine, N-methylglucosamine, triethanolamine and tromethamine; the inorganic bases can be one or more of aluminum hydroxide, calcium hydroxide, potassium hydroxide, sodium carbonate and sodium hydroxide.

The term "isotopic derivative" refers to a compound that differs from the parent compound described herein only in the presence of one or more isotopically enriched atoms. For example, an isotopic derivative has the structure shown by the formula, wherein only hydrogen is replaced by "deuterium" or "tritium", and/or fluorine is replaced by ¹⁸F, and/or carbon is replaced by ¹¹C, ¹³C or ¹⁴C, while the rest part is unchanged. The above isotopic derivatives can be used as analytical tools or probes in biological assays, or as *in vivo* diagnostic imaging tracers for diseases, or as tracers for pharmacodynamics, pharmacokinetics, or receptor studies. Deuterated compounds can often retain comparable activity to non-deuterated compounds, and can achieve better metabolic stability when deuterated at certain specific positions, thereby obtaining certain therapeutic advantages (such as increased half-life *in vivo* or reduced dose requirement). Therefore, the isotopic derivative is preferably a deuterated compound.

The term "solvate" refers to a substance formed from the parent compound described herein and a suitable solvent. The solvent is preferably water or organic solvent.

The term "pharmaceutical composition" refers to a mixture containing a pharmaceutical compound (i.e., one or more of a compound of the formula described herein, a pharmacologically acceptable salt thereof, a tautomer thereof, a stereoisomer thereof, an enantiomer thereof, a diastereoisomer thereof, an isotopic derivative thereof, a crystal form thereof, a solvate thereof, a metabolite thereof, and a racemate containing same), and a pharmaceutically acceptable excipient.

The term "pharmaceutically acceptable excipient" is a pharmaceutically acceptable excipient for delivering the pharmaceutical compound herein to a subject. The pharmaceutical composition may contain 0.1 wt%-99 wt% of pharmaceutical compound depending on the administration method.

The term "subject" refers to a mammal, including, for example, camel, donkey, zebra, cattle, pig, horse, goat, sheep, cat, dog, rat, rabbit, guinea pig, mouse, and primate. **In** some particular embodiments, the subject is a human. **In** some particular embodiments, the subject is a human who is susceptible to, suspected of having, or has suffered from a cancer or bacterial infection.

The term "treatment" refers to eliminating a disease, preventing disease progression, slowing disease progression, reducing the duration of one or more symptoms associated with a disease, improving or reversing at least one measurable parameter associated with a disease, or increasing the survival of subjects with a disease.

The term "effective amount" refers to an amount of the pharmaceutical active ingredient (i.e., the pharmaceutical compound) that elicits the desired effect in a subject. **In** particular embodiments, those skilled in the art can determine the selection of an effective amount based on consideration of a variety of factors (for example, through clinical trials), the factors include the disease to be treated, the symptoms involved, the route of administration, the severity of the disease, the patient's body weight, the patient's immune status, and other factors known to those skilled in the art. The effective amount can be obtained from the dose-response curve derived from an animal model testing system, and it is allowed to be determined according to the opinion of a physician and the situation of each patient. The effective amount of the pharmaceutical compound of the present invention can be 0.5 mg/kg to 500 mg/kg, preferably 1 mg/kg to 200 mg/kg, more preferably 10 mg/kg to 100 mg/kg.

Herein, the same pharmaceutical active ingredient (which refers to a single pharmaceutical compound) or different pharmaceutical active ingredients (which refers to two or more pharmaceutical compounds) can be administered at one time, or can be divided into many lower doses and administered at a certain time interval. It should be understood that the exact dose, duration, and interval of treatment is a function of the disease being treated, and can be determined by inference using animal or clinical trial data. The administration can include a single administration, or two or more administrations at an appropriate time interval. Wherein, the time interval between two adjacent administrations can be 30 minutes, 40 minutes, 50 minutes, 60 minutes, 2 hours, 3 hours, 4 hours, 5 hours, 6 hours, 7 hours, 8 hours, 9 hours, 10 hours, 12 hours, 14 hours, 16 hours, 18 hours, 20 hours, 22 hours, 24 hours, one and a half days, 2 days, 3 days, 4 days, 5 days, 6 days, 7 days, 8 days, 9 days, 10 days, 1 week, 2 weeks, 3 weeks, 4 weeks, 5 weeks, 6 weeks, 1 month, 2 months, 3 months , 4 months, 5 months, 6 months, 7 months, 8 months, 9 months, 10 months, 11 months or 12 months.

Each pharmaceutical active ingredient (each pharmaceutical compound) mentioned herein can be used as the only active compound, or can be administered in combination with other active compounds (which refers to compounds other than the pharmaceutical compound described herein), as long as they do not produce other adverse effects, for example, allergic responses. "Combined administration" includes simultaneous or sequential administration of each active compound.

The term "combined administration" refers to a method of providing two or more active compounds simultaneously or sequentially to a subject for therapeutic purposes. When "combined administration" is involved, the time interval between each administration is sufficient to achieve synergistic effect between each active compound administered.

When the term "about" is applied to a parameter such as weight, volume, pH, concentration, temperature, etc., it is indicated that the parameter can vary ± 10%, and sometimes more preferably within ±5%. As will be understood by those skilled in the art, when a parameter is not critical, the value is usually given for illustrative purposes only, but not as limitation.

### DETAILED DESCRIPTION OF THE INVENTION

Those skilled in the art will better understand the present invention by reading the following examples. These examples are only used to illustrate the present invention, and do not limit the scope of the present invention.

The compound of the present invention is prepared by using convenient starting materials and general preparation procedures. The present invention provides typical or preferential reaction conditions, such as reaction temperature, time, solvent, pressure, and molar ratio of reactants. However, unless otherwise specified, other reaction conditions can also be adopted. Optimal conditions may vary with the use of specific reactants or solvents, but under normal circumstances, reaction optimization steps and conditions can be determined.

In addition, some protecting groups may be used in the present invention to protect certain functional groups from unnecessary reactions. The protecting groups suitable for various functional groups and their protection or deprotection conditions are well known to those skilled in the art. For example, "Protective Groups In Organic Synthesis" by T. W. Greene and G. M. Wuts (3rd edition, Wiley, New York, 1999 and citations in the book) describes the protection or deprotection of a large number of protecting groups in detail.

The separation and purification of compounds and intermediates adopt appropriate methods and steps according to specific needs, for example, filtration, extraction, distillation, crystallization, column chromatography, preparative thin-layer chromatography, preparative high performance liquid chromatography, or a combination of the above methods. The examples described in the present invention may be referred to for the specific method used. Of course, other similar separation and purification methods can also be used. They can be characterized using conventional methods (including physical constants and spectral data).

The method of purity analysis is as follows: gradient elution is performed with a Kinetex EVO C18 (50×4.6 mm, 5 µm, 100Å) column and with acetonitrile-water as the mobile phase at a flow rate of 1.5 mL/min and a detection wavelength of 220 nm.

MS determination is performed using a LC (Agilent 1260 Infinity II)/MS (G6125B single quadrupole) mass spectrometer (manufacturer: Agilent) (Photodiode Array Detector).

The structure of compounds is determined by hydrogen nuclear magnetic resonance with a model WNMR-I-400MHz device.

Preparative liquid chromatography is performed using an Agilent 1260 Infinity II high performance liquid chromatograph (manufacturer: Agilent), with a Daisogel C18 10 µm 100A (30 mm×250 mm) column and with acetonitrile/water as the mobile phase.

Thin layer chromatography (TLC) is performed using Qingdao Haiyang Chemical GF254 silica gel plates, 0.20 mm-0.25 mm silica gel plates are used for thin layer chromatography for reaction monitoring, and 0.5 mm silica gel plates are used for thin layer chromatography for separation and purification.

Silica gel column chromatography is performed with Qingdao Haiyang silica gel of 100~200 mesh, 200~300 mesh and 300~400 mesh as carriers.

The known starting materials of the present invention may be synthesized by or according to methods known in the art, or may be purchased from WHmall, Beijing Ouhe, Sigma, J&K Scientific, Yishiming, Shanghai Shuya, Shanghai InnoChem, Energy Chemical, Shanghai Bide and other companies.

Unless otherwise specified in the examples, the reactions are all carried out under nitrogen atmosphere.

Nitrogen atmosphere means that a reaction flask is equipped with a nitrogen balloon with a volume of about 1 L.

A reaction solvent, an organic solvent or an inert solvent respectively means that the solvent used does not participate in a reaction under the described reaction conditions, and it includes, for example, benzene, toluene, acetonitrile, tetrahydrofuran (THF), dimethylformamide (DMF), chloroform, dichloromethane, ether, methanol, and N-methylpyrrolidone (NMP).

Unless specified in the examples, a solution refers to an aqueous solution.

Unless otherwise defined, all professional and scientific terms used herein have the same meaning as those familiar to those skilled in the art. In addition, any methods and materials similar or equivalent to the content described herein can be applied to the method of the present invention.

Unless otherwise specified, the mixing ratio of different solvents is volume ratio.

In the following examples, the abbreviations involved have the following meanings:
NBS: N-bromosuccinimide;
NCS: N-chlorosuccinimide;
NIS: N-iodosuccinimide;
DPPF: 1,1'-bis(diphenylphosphino)ferrocene.

### Example 1

### Synthesis of 5-bromo-7-cyano-8-hydroxyquinoline (1)

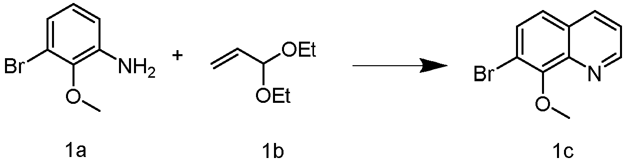

2-Methoxy-3-bromoaniline (Compound 1a) (5.7 g, 28.5 mmol, 1 eq) was dissolved in 100 mL of 6 M aqueous hydrochloric acid solution and the mixture was heated to reflux. 3,3-Diethoxyprop-1-ene (Compound 1b) (11.1 g, 85.5 mmol, 3 eq) was added dropwise and the reaction was carried out for 2 hours. The reaction solution was cooled, the pH was adjusted to neutral with aqueous sodium carbonate solution, and the reaction solution was extracted with 30 mL×2 dichloromethane. The organic phases were combined and concentrated to obtain a crude product, which was purified by silica gel column chromatography (eluent: PE:EA=10:1~1:1) to obtain 800 mg of 7-bromo-8-methoxyquinoline (Compound 1c) as a colorless oil with a purity of 90%.

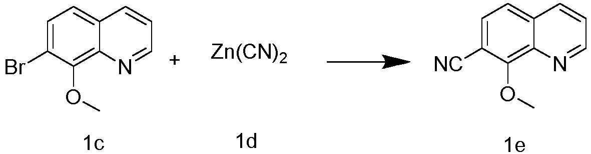

7-Bromo-8-methoxyquinoline (Compound 1c) (400 mg, 1.18 mmol, 1 eq) was dissolved in 5 mL of anhydrous dimethylformamide, and zinc cyanide (Compound 1d) (110.5 mg, 0.95 mmol, 0.8 eq) and tetrakis(triphenylphosphine)palladium (68 mg, 0.059 mmol, 0.05 eq) were added. The reaction system was purged with nitrogen, and the reaction was carried out at 110°C for 8 hours. The reaction solution was concentrated to obtain a crude product, which was purified by silica gel column chromatography (eluent: PE:EA=10:1~0:1) to obtain 130 mg of 7-cyano-8-methoxyquinoline (Compound 1e) as a white solid with a purity of 90%.

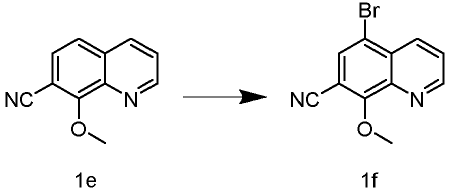

7-Cyano-8-methoxyquinoline (Compound 1e) (120 mg, 1.43 mmol, 1 eq) was dissolved in 5 mL of anhydrous dimethylformamide, and NBS (506 mg, 2.86 mmol, 2 eq) was added. The reaction was carried out at room temperature for 8 hours and 20 mL of water was added. The precipitated solid was filtered and dried to obtain 100 mg of 5-bromo-7-cyano-8-methoxyquinoline (Compound 1f) as a white solid with a purity of 90%.

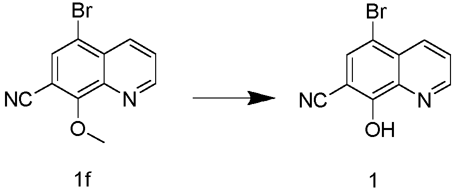

5-Bromo-7-cyano-8-methoxyquinoline (Compound 1f) (100 mg, 0.38 mmol, 1 eq) was dissolved in 5 mL of dimethylformamide and 200 mg of lithium chloride was added. The reaction was carried out at 80°C for 1 hour and 20 mL of water was added. The pH was adjusted to neutral and the reaction solution was extracted with 5 mL×2 dichloromethane. The organic phases were combined, and the crude product was purified by pre-HPLC to obtain 8.8 mg of 5-bromo-7-cyano-8-hydroxyquinoline (Compound 1) as a white solid with a purity of 97%.

MS calculated: 247.96, 249.96; MS found: 249.0, 250.9 [M+H]⁺.

¹H NMR (400 MHz, DMSO-d6): δ 9.10 (d, J=4Hz,1H), 8.56 (d, J=8Hz,1H), 8.13 (s, 1H), 7.93 (dd, J=4Hz, J=8Hz,1H).

### Example 2

### Synthesis of 5-trifluoromethyl-7-cyano-8-hydroxyquinoline (2)

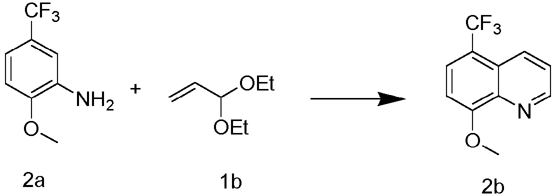

2-Methoxy-5-trifluoromethylaniline (Compound 2a) (5 g, 26.16 mmol, 1 eq) was dissolved in 50 mL of 3 M aqueous hydrochloric acid solution and heated to reflux. 3,3-Diethoxyprop-1-ene (Compound 1b) (17.1 g, 130.8 mmol, 5 eq) was added dropwise and the reaction was carried out for 3 hours. The reaction solution was cooled, the pH was adjusted to neutral with aqueous sodium carbonate solution, and the reaction solution was extracted with 50 mL×2 dichloromethane. The organic phases were combined and concentrated to obtain a crude product, which was purified by silica gel column chromatography (eluent: PE:EA=10:1~0:1) to obtain 3.1 g of 5-trifluoromethyl-8-methoxyquinoline (Compound 2b) as a colorless oil with a purity of 90%.

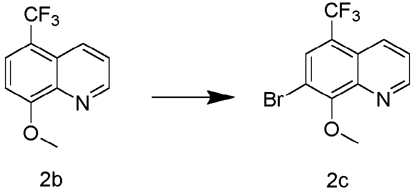

5-Trifluoromethyl-8-methoxyquinoline (Compound 2b) (3.1 g, 13.6 mmol, 1 eq) was dissolved in 30 mL of dimethylformamide and NBS (4.84 g, 27.2 mmol, 2 eq) was added. The reaction was carried out at room temperature for 3 hours. The reaction solution was diluted with 50 mL of water, extracted with 50 mL of dichloromethane and separated into two phases. The organic phase was concentrated and the crude product was purified by silica gel column chromatography (eluent: PE:EA=10:1~0:1) to obtain 1.2 g of 5-trifluoromethyl-7-bromo-8-methoxyquinoline (Compound 2c) as a light red liquid with a purity of 90%.

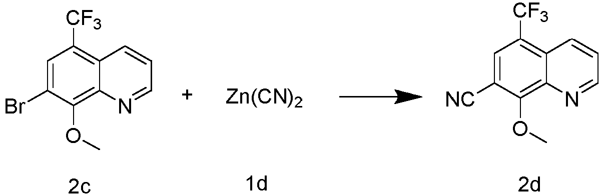

5-Trifluoromethyl-7-bromo-8-methoxyquinoline (Compound 2c) (1.2 g, 3.92 mmol, 1 eq) was dissolved in 20 mL of anhydrous dimethylformamide, and zinc cyanide (Compound 1d) (917.3 mg, 7.84 mmol, 2 eq) and tetrakis(triphenylphosphine)palladium (226 mg, 0.196 mmol, 0.05 eq) were added. The reaction system was purged with nitrogen and the reaction was carried out at 110°C for 8 hours. The reaction solution was diluted with 50 mL of water and extracted with 25 mL×2 dichloromethane. The organic phases were combined, dried over anhydrous sodium sulfate, filtered, and concentrated to obtain 900 mg of crude 5-trifluoromethyl-7-cyano-8-methoxyquinoline (Compound 2d).

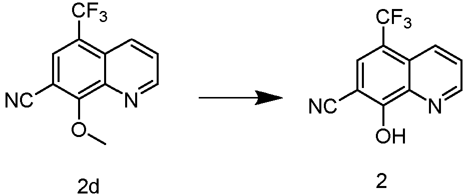

5-Trifluoromethyl-7-cyano-8-methoxyquinoline (Compound 2d) (900 mg, 3.58 mmol, 1 eq) was dissolved in 10 mL of dimethylformamide and 767 mg of lithium chloride was added. The reaction was carried out at 80°C for 2 hours and 50 mL of water was added. The pH was adjusted to neutral and the reaction solution was extracted with 25 mL×2 dichloromethane. The organic phases were combined and concentrated to obtain a crude product, which was purified by silica gel column chromatography (eluent: PE:EA=10:1~0:1) to obtain 220 mg of product (with a purity of 80%). The product was purified again by pre-HPLC to obtain 32 mg of 5-trifluoromethyl-7-cyano-8-hydroxyquinoline (Compound 2) as a white solid with a purity of 96%.

MS calculated: 238.04; MS found: 239.0 [M+H]⁺.

¹H NMR (400 MHz, DMSO-d6): δ 9.13 (d, J=4Hz,1H), 8.50 (d, J=8Hz,1H), 8.22 (s, 1H), 7.99 (dd, J=4Hz, J=8Hz,1H).

### Example 3

### Synthesis of 4-methyl-7-cyano-8-hydroxyquinoline (3)

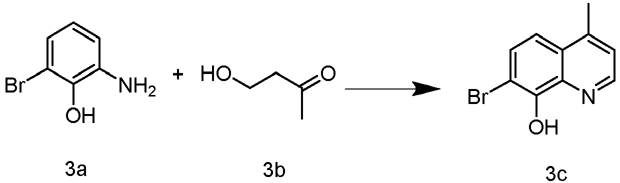

2-Amino-6-bromophenol (Compound 3a) (5 g, 26.6 mmol, 1 eq) was dissolved in 100 mL of 1 M aqueous hydrochloric acid solution and heated to reflux. 4-Hydroxy-2-butanone (Compound 3b) (7 g, 79.8 mmol, 3 eq) was added dropwise and the reaction was carried out for 2 hours. The reaction solution was cooled and the pH was adjusted to neutral with aqueous sodium carbonate solution. The reaction solution was extracted with 50 mL×2 ethyl acetate and concentrated to obtain 7 g of crude 4-methyl-7-bromo-8-hydroxyquinoline (Compound 3c) as a red solid.

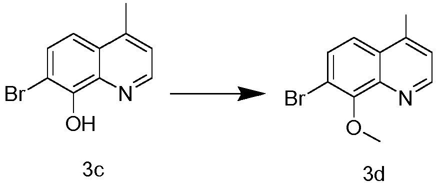

4-Methyl-7-bromo-8-hydroxyquinoline (Compound 3c) (7 g, 29.2 mmol, 1 eq) was dissolved in 70 mL of solution of dimethylformamide, and potassium carbonate (6 g, 43.8 mmol, 1.5 eq) and methyl iodide (6.3 g, 43.8 mmol, 1.5 eq) were added. The reaction was carried out at room temperature for 3 hours. The reaction solution was diluted with 150 mL of water, extracted with 50 mL×2 ethyl acetate and separated into two phases. The organic phases were combined, dried over anhydrous sodium sulfate, filtered, and concentrated to obtain a crude product, which was purified by silica gel column chromatography (eluent: PE/EA=10:1~1:1) to obtain 2 g of product 4-methyl-7-bromo-8-methoxyquinoline (Compound 3d) as a red liquid.

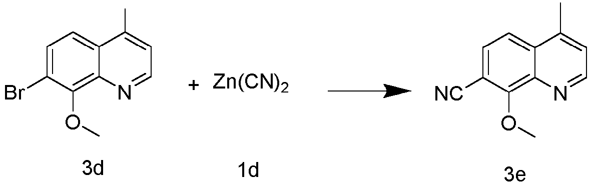

4-Methyl-7-bromo-8-methoxyquinoline (Compound 3d) (2 g, 7.88 mmol, 1 eq), zinc cyanide (Compound 1d) (900 mg, 3.94 mmol, 0.5 eq), Pd₂(dba)₃ (226 mg, 0.394 mmol, 0.05 eq) and DPPF (220 mg, 0.394 mmol, 0.05 eq) were dissolved in 20 mL of dimethylformamide. The reaction system was purged with nitrogen and the reaction was carried out at 100°C for 10 hours. The reaction solution was diluted with 50 mL of water, extracted with 20 mL×2 ethyl acetate and separated into two phases. The organic phases were combined, dried over anhydrous sodium sulfate, filtered, and the filtrate was concentrated to obtain a crude product, which was purified by silica gel column chromatography (eluent: PE:EA=10:1~1:1) to obtain 800 mg of 4-methyl-7-cyano-8-methoxyquinoline (Compound 3e) as a red oil with a purity of 80%.

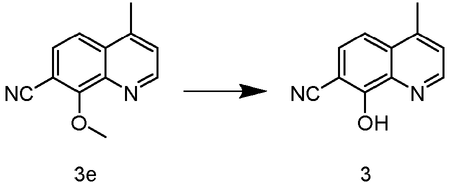

4-Methyl-7-cyano-8-methoxyquinoline (Compound 3e) (800 mg, 4 mmol, 1 eq) was dissolved in 10 mL of dimethylformamide and 200 mg of lithium chloride was added. The reaction was carried out at 100°C for 2 hours and 50 mL of water was added. The pH was adjusted to neutral and the product was precipitated and filtered and dried to obtain a crude product, which was triturated twice with 20 mL of methanol/15 mL of dichloromethane to obtain 23.7 mg of 4-methyl-7-cyano-8-hydroxyquinoline (Compound 3) as a white-gray solid with a purity of 98%.

MS calculated: 184.06; MS found: 185.1 [M+H]⁺.

¹H NMR (400 MHz, DMSO-d6): δ8.86 (d,J=8Hz,1H), 7.69 (d,J=12Hz,1H),7.63 (dd, J=4Hz, 1H), 7.58 (dd, J=8Hz,1H),2.71(s,3H).

### Example 4

### Synthesis of 2-fluoro-7-cyano-8-hydroxyquinoline (4)

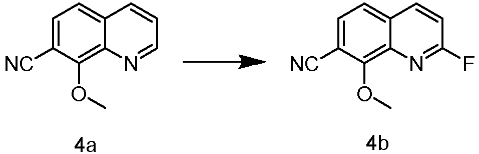

7-Cyano-8-methoxyquinoline (Compound 4a) (150 mg, 0.815 mmol, 1 eq) was dissolved in 17 mL of anhydrous acetonitrile and silver difluoride (708 mg, 4.8 mmol, 6 eq) was added. The reaction was carried out at room temperature for 1 hour and the reaction solution was filtered. The filtrate was concentrated to obtain 100 mg of crude 2-fluoro-7 cyano-8-methoxyquinoline (Compound 4b).

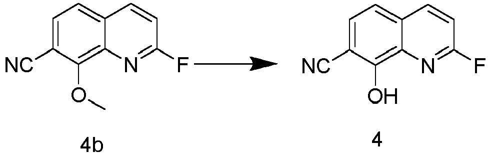

2-Fluoro-7-cyano-8-methoxyquinoline (Compound 4b) (100 mg, 0.54 mmol, 1 eq) was dissolved in 5 mL of dimethylformamide and 228 mg of lithium chloride was added. The reaction was carried out at 80°C for 2 hours and the reaction solution was concentrated. The resulting crude product was purified by pre-HPLC to obtain 12 mg of 2-fluoro-7-cyano-8-hydroxyquinoline (Compound 4) as a yellow-green solid with a purity of 96%.

MS calculated: 188.0; MS found: 189.1 [M+H]⁺.

¹H NMR (400 MHz, DMSO-d6): δ8.30~8.37 (m,1H), 7.70~7.75 (m,1H), 7.63 (dd, J=4Hz, J=8Hz,1H), 7.37 (dd, J=8Hz,1H).

### Example 5

### Synthesis of 4-methyl-5-chloro-7-cyano-8-hydroxyquinoline (5)

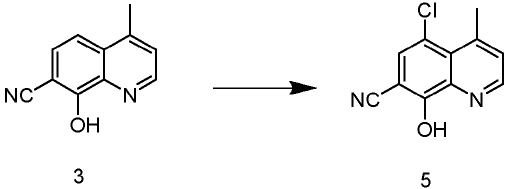

4-Methyl-7-cyano-8-hydroxyquinoline (Compound 3) (300 mg, 1.62 mmol, 1 eq) was dissolved in 10 mL of dimethylformamide and NCS (425 mg, 3.2 mmol, 2 eq) was added. The reaction was carried out at room temperature for 30 min. The reaction solution was diluted with 30 mL of water and extracted with 15 mL×2 dichloromethane. The organic phases were combined, dried and filtered, and the filtrate was concentrated. The resulting crude product was purified by pre-HPLC to obtain 155 mg of 4-methyl-5-bromo-7-cyano-8-hydroxyquinoline (Compound 5) as a light yellow solid with a purity of 98%.

MS calculated: 218.02; MS found: 219.1 [M+H]⁺.

¹H NMR (400 MHz, DMSO-d6): δ8.85 (d, J=4Hz,1H), 7.84 (s, 1H), 7.66 (d, J=8Hz, 1H), 3.01(s,3H).

### Example 6

### Synthesis of 4-methyl-5-bromo-7-cyano-8-hydroxyquinoline (6)

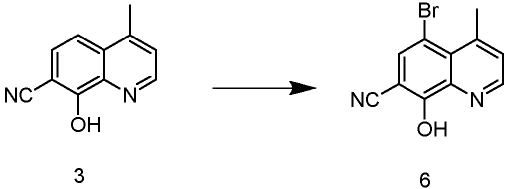

4-Methyl-7-cyano-8-hydroxyquinoline (Compound 3) (300 mg, 1.62 mmol, 1 eq) was dissolved in 10 mL of dimethylformamide and NBS (569 mg, 3.2 mmol, 2 eq) was added. The reaction was carried out at room temperature for 30 min. The reaction solution was diluted with 50 mL of water and extracted with 15 mL×2 dichloromethane. The organic phases were combined, dried and filtered, and the filtrate was concentrated. The resulting crude product was triturated with 30 mL of methanol/5 mL of dichloromethane to obtain 105 mg of 4-methyl-5-bromo-7-cyano-8-hydroxyquinoline (Compound 6) as a light yellow solid with a purity of 97%.

MS calculated: 261.97, 263.97; MS found: 263.0, 265.0 [M+H]⁺.

¹H NMR (400 MHz, DMSO-d6): δ8.85 (d, J=4Hz,1H), 8.06 (s, 1H), 7.69 (d, J=8Hz, 1H), 3.01(s,3H).

### Example 7

### Synthesis of 5,6-dichloro-7-cyano-8-hydroxyquinoline (7)

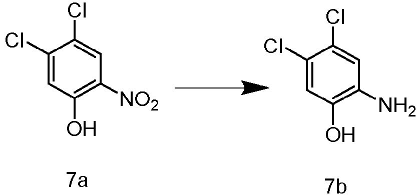

5 g of 2-nitro-4,5-dichlorophenol (Compound 7a) was dissolved in 50 mL of tetrahydrofuran and 500 mg of wetted palladium on carbon was added. The reaction system was equipped with a hydrogen balloon and the reaction was carried out for 8 hours. The reaction solution was filtered and the filtrate was concentrated to obtain 4.7 g of product 2-amino-4,5-dichlorophenol (Compound 7b).

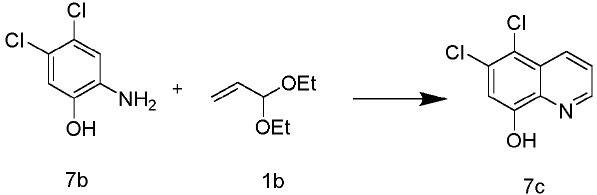

2-Amino-4,5-dichlorophenol (Compound 7b) (4.7 g, 26.5 mmol, 1 eq) was dissolved in 100 mL of 1 M aqueous hydrochloric acid solution and heated to reflux. 3,3-Diethoxyprop-1-ene (Compound 1b) (10 g, 79.7 mmol, 3 eq) was added dropwise and the reaction was carried out for 1 hour. The reaction solution was cooled, the pH was adjusted to neutral with aqueous sodium carbonate solution, and the reaction solution was extracted with 30 mL×2 dichloromethane. The organic phases were combined and concentrated to obtain 3.5 g of crude 5,6-dichloro-8-hydroxyquinoline (Compound 7c) as a black solid.

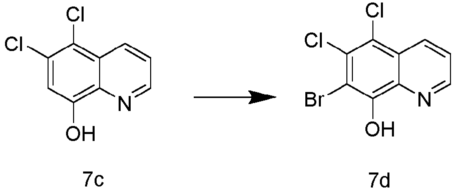

5,6-Dichloro-8-hydroxyquinoline (Compound 7c) (3.5 g, 16.3 mmol, 1 eq) was dissolved in 50 mL of dimethylformamide and NBS (2.9 g, 16.3 mmol, 1.0 eq) was added. The reaction was carried out at room temperature for 2 hours. The reaction solution was diluted with 100 mL of water, extracted with 50 mL×2 dichloromethane and separated into two phases. The organic phases were combined, dried, and then filtered and concentrated to obtain 4 g of crude 5,6-dichloro-7-bromo 8-hydroxyquinoline (Compound 7d) as a yellow solid.

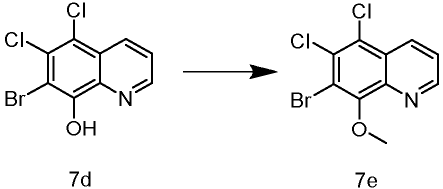

5,6-Dichloro-7-bromo 8-hydroxyquinoline (Compound 7d) (4 g, 13.7 mmol, 1 eq) was dissolved in 40 mL of solution of dimethylformamide, and potassium carbonate (1.89 g, 13.6 mmol, 1 eq) and methyl iodide (1.94 g, 13.6 mmol, 1 eq) were added. The reaction was carried out at room temperature for 3 hours. The reaction solution was diluted with 150 mL of water, extracted with 50 mL×2 dichloromethane and separated into two phases. The organic phases were combined, dried over anhydrous sodium sulfate and filtered. The filtrate was concentrated to obtain a crude product, which was purified by silica gel column chromatography (eluent: PE/EA=10:1~2:1) to obtain 400 mg of product 5,6-dichloro-7-bromo-8-methoxyquinoline (Compound 7e) as a light yellow solid with a purity of 90%.

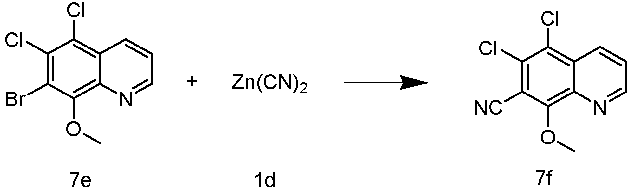

5,6-Dichloro-7-bromo-8-methoxyquinoline (Compound 7e) (400 mg, 1.31 mmol, 1 eq), zinc cyanide (Compound 1d) (75 mg, 0.65 mmol, 0.5 eq), Pd₂(dba)₃ (75 mg, 0.13 mmol, 0.1 eq) and DPPF (72 mg, 0.13 mmol, 0.1 eq) were dissolved in 10 mL of dimethylformamide. The reaction system was purged with nitrogen and the reaction was carried out at 90°C for 8 hours. The reaction solution was diluted with 50 mL of water, extracted with 30 mL×2 ethyl acetate and separated into two phases. The organic phases were combined, dried over anhydrous sodium sulfate and filtered. The filtrate was concentrated to obtain a crude product, which was purified by silica gel column chromatography (eluent: PE:EA=10:1~1:1) to obtain 200 mg of crude 5,6-dichloro-7-cyano-8-methoxyquinoline (Compound 7f) as a white solid.

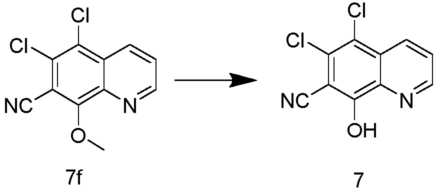

5,6-Dichloro-7-cyano-8-methoxyquinoline (Compound 7f) (200 mg, 0.8 mmol, 1 eq) was dissolved in 3 mL of dimethylformamide and 100 mg of lithium chloride was added. The reaction was carried out at 100°C for 1 hour and the reaction solution was concentrated. The resulting crude product was purified by pre-HPLC to obtain 36 mg of product 5,6-dichloro-7-cyano-8-hydroxyquinoline (Compound 7) as a white solid with a purity of 98%.

MS calculated: 237.97; MS found: 239.0 [M+H]⁺.

¹H NMR (400 MHz, DMSO-d6): δ9.10(d,J=4Hz, 1H),8.62 (d,J=8Hz, 1H), 7.97(dd,J=4Hz,J=8Hz, 1H).

### Example 8

### Synthesis of 7-cyano-8-hydroxyquinoline-5-carboxylic acid (8)

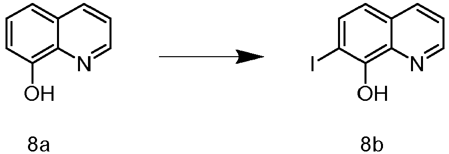

8-Hydroxyquinoline (Compound 8a) (10 g, 68.6 mmol, 1 eq) and NIS (18.6 g, 82.6 mmol, 1.2 eq) were dissolved in 200 mL of chloroform. The reaction was carried out at 40°C for 20 hours and the reaction solution was filtered. 100 mL of filtrate was washed twice with 5% aqueous sodium thiosulfate solution and separated into two phases. The organic phase was concentrated to obtain a crude product, which was triturated with 50 mL of methanol/50 mL of water and filtered. The filter cake was dried to obtain 12 g of product 7-iodo-8-hydroxyquinoline (Compound 8b).

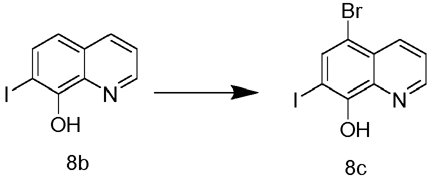

7-Iodo-8-hydroxyquinoline (Compound 8b) (12 g, 44.3 mmol, 1 eq) was dissolved in 180 mL of dimethylformamide and NBS (9.4 g, 55.3 mmol, 1.2 eq) was added. The reaction was carried out at room temperature for 1 hour and the reaction solution was filtered. The filter cake was washed with water and dried to obtain 13 g of crude 5-bromo-7-iodo-8-hydroxyquinoline (Compound 8c) as a yellow solid.

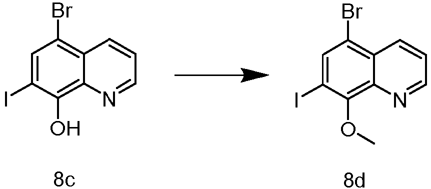

5-Bromo-7-iodo-8-hydroxyquinoline (Compound 8c) (13 g, 37.2 mmol, 1 eq) was dissolved in 150 mL of solution of dimethylformamide, and potassium carbonate (7.7 g, 55.8 mmol, 1.5 eq) and methyl iodide (7.7 g, 44.7 mmol, 1.2 eq) were added. The reaction was carried out at room temperature for 2 hours. The reaction solution was diluted with 200 mL of water, extracted with 50 mL×2 dichloromethane and separated into two phases. The organic phases were combined, dried over anhydrous sodium sulfate and filtered. The filtrate was concentrated to obtain a crude product, which was purified twice by silica gel column chromatography (eluent: PE/EA=10:1~1:1) to obtain 3.7 g of product 5-bromo-7-iodo-8-methoxyquinoline (Compound 8d) as a light yellow solid.

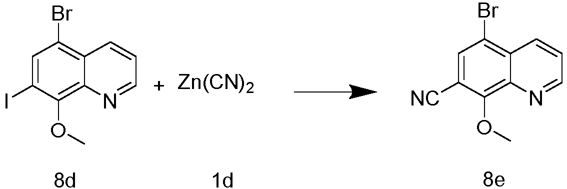

5-Bromo-7-iodo-8-methoxyquinoline (Compound 8d) (3.7 g, 10.2 mmol, 1 eq), zinc cyanide (Compound 1d) (599 mg, 5.1 mmol, 0.5 eq), Pd₂(dba)₃ (281 mg, 0.51 mmol, 0.05 eq) and DPPF (276 mg, 0.51 mmol, 0.05 eq) were dissolved in 40 mL of dimethylformamide. The reaction system was purged with nitrogen, and the reaction was carried out at 100°C for 10 hours. The reaction solution was diluted with 50 mL of water, extracted with 20 mL×2 ethyl acetate and separated into two phases. The organic phases were combined, dried over anhydrous sodium sulfate and filtered. The filtrate was concentrated to obtain a crude product, which was purified by silica gel column chromatography (eluent: PE:EA=10:1~1:1) to obtain 1.4 g of product 5-bromo-7-cyano-8-methoxyquinoline (Compound 8e) as a white solid with a purity of 90%.

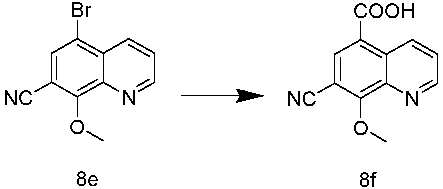

Compound 8e (200 mg, 0.763 mmol, 1 eq) was dissolved in 5 mL of anhydrous tetrahydrofuran. The reaction mixture was purged with nitrogen and cooled to -78°C. n-Butyllithium (1 M, 1 mmol, 1.3 eq) was added and the mixture was stirred for 10 minutes. Small pieces of dry ice were added and the reaction was carried out for 5 minutes. The reaction was quenched by the addition of 2 mL of water, and the reaction solution was concentrated to obtain 250 mg of crude 7-cyano-8-methoxyquinoline-5-carboxylic acid (Compound 8f).

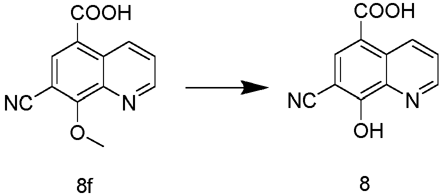

7-Cyano-8-methoxyquinoline-5-carboxylic acid (Compound 8f) (200 mg, 0.93 mmol, 1 eq) was dissolved in 3 mL of dimethylformamide and 100 mg of lithium chloride was added. The reaction was carried out at 100°C for 2 hours and the reaction solution was concentrated. The resulting crude product was purified by pre-HPLC to obtain 40 mg of product 7-cyano-8-hydroxyquinoline-5-carboxylic acid (Compound 8) as a yellow solid with a purity of 98%.

MS calculated: 214.18; MS found: 215.1 MS [M+H]⁺.

¹H NMR (400 MHz, DMSO-d6): δ 10.1(s, 1H), 9.58 (d,J=4Hz,1H),9.27 (d,J=4Hz,1H), 7.86(dd, J=4Hz, J=8Hz,1H), 7.71(s,1H).

### Example 9

### Synthesis of 5-chloro-7-cyano-8-hydroxyquinoline (9)

The NBS in Example 1 was replaced by NCS in equimolar ratio, and the other reaction steps were the same as in Example 1, 5-chloro-7-cyano-8-hydroxyquinoline (Compound 9) was obtained accordingly.

MS calculated: 184.06; MS found: 185.1 [M+H]⁺.

¹H NMR (400 MHz, DMSO-d6): δ 8.67 (d, J= 4.3 Hz, 1H), 8.34 (dd, J = 8.4, 1.5 Hz), 7.68 (dd, 8.4, 4.3 Hz, 1H), 7.44 (s, 1H).

### Biological Assay

Test Example 1. Assay of inhibitory activity of the compounds of the present invention on bacteria

### 1. Test drug

The compounds of the Examples of the present invention

### 2. Test bacteria

The test bacteria were 2 strains of carbapenem-resistant *Escherichia coli* (18-W09-093 and 18-W18-070), 2 strains of carbapenem-resistant *Acinetobacter baumannii* (18-W15-036 and 18-W13-081), 1 strain of methicillin-resistant *Staphylococcus aureus* (19-W03-012) and 1 strain of methicillin-sensitive *Staphylococcus aureus* (19-W01-012), isolated clinically, with duplicate strains isolated from the same patient being excluded. Besides, there were 3 quality control strains, namely *Escherichia coli* ATCC 25922, *Acinetobacter baumannii* ATCC 19606, and *Staphylococcus aureus* ATCC 29213. The above strains were all provided by Shanghai GenoMatrix Medical Laboratory Co., Ltd.

### 3. Test methods for drug sensitivity

The MIC of the compound of the present invention on clinically isolated strains was assayed by the broth microdilution method according to the relevant documents from the Clinical and Laboratory Standards Institute (CLSI) of United States.

3.1 Preparation of antimicrobial drugs: the compounds of the present invention were all dissolved with DMSO. The drug concentration ranged from 128 mg/L to 0.06 mg/L.

3.2 Culture medium: cation adjusted Mueller-Hinton broth (CAMHB) was used.

3.3 Inoculation amount of bacteria: the isolated pure culture of test bacteria cultured overnight was adjusted to 0.5 McFarland turbidity by direct colony suspension and diluted 100 folds, and the final inoculation amount was 10⁵ CFU/mL.

3.4 Culture conditions: the bacteria were cultured in air at 35±2°C for 20 hours.

### 4. Reading and interpretation of results

The results of the drug sensitivity test were interpretated according to the standards of CLSI M100-ED29:2019.

### 5. Result statistics

The statistical analysis of the results of the drug sensitivity test was done by using WHONET version 5.6 software.

The inhibitory MIC values of the compounds of the present invention on various bacteria were shown in Table 1 below.

**Table 1. The inhibitory MIC values of the compounds of the present invention on bacteria**

| Compound No. | *Escherichia coli* | | | *Acinetobacter baumannii* | | | *Staphylococcus aureus* | | |
|---|---|---|---|---|---|---|---|---|---|
| | 18-W09-093 | 18-W18-070 | ATCC2 5922 | 18-W15-036 | 18-W13-081 | ATCC19 606 | 19-W03-012 | 19-W01-012 | ATCC2 9213 |
| 1 | 32 | 32 | 8 | 16 | 4 | 4 | 4 | 4 | 4 |
| 2 | 64 | 64 | 32 | 64 | 32 | 16 | 4 | 4 | 8 |
| 3 | 16 | 16 | 8 | 8 | 8 | 8 | 8 | 8 | 8 |
| 4 | 16 | 16 | 8 | 8 | 8 | 4 | 16 | 16 | 16 |
| 5 | 32 | 16 | 8 | 8 | 2 | 4 | 2 | 4 | 4 |
| 6 | 32 | 32 | 8 | 32 | 4 | 4 | 2 | 4 | 4 |
| 7 | >64 | >64 | 32 | 32 | 4 | 2 | 0.5 | 0.5 | 1 |
| 8 | >64 | >64 | >64 | >64 | >64 | >64 | >64 | >64 | >64 |
| 9 | 32 | 32 | 16 | 16 | 8 | 4 | 8 | 8 | 8 |
| A | 8 | 16 | 8 | 8 | 4 | 4 | 4 | 4 | 4 |

wherein Compound A is 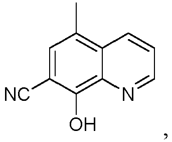 synthesized according to literature (Fiedler, H. (1960). Synthese von Methyl-8-hydroxy-chinolin-aldehyden. Archiv Der Pharmazie, 293(6), 609-621. doi:10.1002/ardp.19602930606).

From the data in the above table, it can be seen that the compounds of the present invention have excellent antibacterial activity, particularly on gram-negative bacteria such as *Escherichia coli, Acinetobacter baumannii,* and gram-positive bacteria such as *Staphylococcus aureus.* Therefore, the compounds of the present invention can treat infectious diseases caused by gram-negative bacteria or gram-positive bacteria.

Although the particular embodiments of the present invention are described above, those skilled in the art should understand that they are only exemplary, and that the protection scope of the present invention is defined by the appended claims. Those skilled in the art may make various changes or modifications to these embodiments without deviating from the principle and essence of the present invention, but these changes and modifications all fall within the protection scope of the present invention.

## Claims

1. A compound of formula (I), a stereoisomer thereof, or a pharmaceutically acceptable salt thereof: 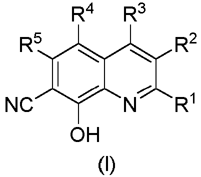 wherein:
R¹ is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R² is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R³ is selected from the group consisting of hydrogen atom, alkyl, halogen, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R⁴ is selected from the group consisting of hydrogen atom, halogen, haloalkyl, carboxy, hydroxy, hydroxyalkyl, alkoxy, haloalkoxy, amino, thiol, alkenyl, alkynyl, cycloalkyl, heterocyclyl, aryl and heteroaryl;
R⁵ is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
provided that R¹ to R⁵ are not all hydrogen atoms at the same time.

2. The compound of formula (I), the stereoisomer thereof, or the pharmaceutically acceptable salt thereof according to claim 1, wherein R⁴ is selected from the group consisting of hydrogen atom, halogen, haloC₁₋₆ alkyl, carboxy and hydroxy, preferably selected from the group consisting of hydrogen atom, halogen, haloC₁₋₆ alkyl and carboxy, more preferably selected from the group consisting of hydrogen atom, bromine atom, chlorine atom, trifluoromethyl and carboxy.

3. The compound of formula (I), the stereoisomer thereof, or the pharmaceutically acceptable salt thereof according to claim 1 or 2, wherein R³ is selected from the group consisting of hydrogen atom, C₁₋₆ alkyl, halogen and hydroxy, preferably hydrogen atom or C₁₋₆ alkyl, more preferably hydrogen atom or methyl.

4. The compound of formula (I), the stereoisomer thereof, or the pharmaceutically acceptable salt thereof according to any one of claims 1 to 3, wherein R¹ is selected from the group consisting of hydrogen atom, halogen, C₁₋₆ alkyl and hydroxy, preferably hydrogen atom or halogen, more preferably hydrogen atom or fluorine atom.

5. The compound of formula (I), the stereoisomer thereof, or the pharmaceutically acceptable salt thereof according to any one of claims 1 to 4, wherein R⁵ is selected from the group consisting of hydrogen atom, halogen, alkyl and hydroxy, preferably hydrogen atom or halogen, more preferably hydrogen atom or chlorine atom.

6. The compound of formula (I), the stereoisomer thereof, or the pharmaceutically acceptable salt thereof according to any one of claims 1 to 5, wherein R² is selected from the group consisting of hydrogen atom, halogen, C₁₋₆ alkyl and hydroxy, preferably hydrogen atom.

7. The compound of formula (I), the stereoisomer thereof, or the pharmaceutically acceptable salt thereof according to any one of claims 1 to 6, being a compound of formula (II), a stereoisomer thereof, or a pharmaceutically acceptable salt thereof: 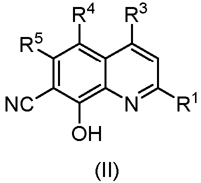 wherein:
R¹ is hydrogen atom or halogen, preferably hydrogen atom or fluorine atom;
R³ is hydrogen atom or C₁₋₆ alkyl, preferably hydrogen atom or methyl;
R⁴ is selected from the group consisting of hydrogen atom, halogen, haloC₁₋₆ alkyl and carboxy, preferably selected from the group consisting of hydrogen atom, bromine atom, chlorine atom, trifluoromethyl and carboxy;
R⁵ is hydrogen atom or halogen, preferably hydrogen atom or chlorine atom;
provided that R¹ to R⁵ are not all hydrogen atoms at the same time.

8. The compound of formula (I), the stereoisomer thereof, or the pharmaceutically acceptable salt thereof according to any one of claims 1 to 7, selected from the group consisting of:

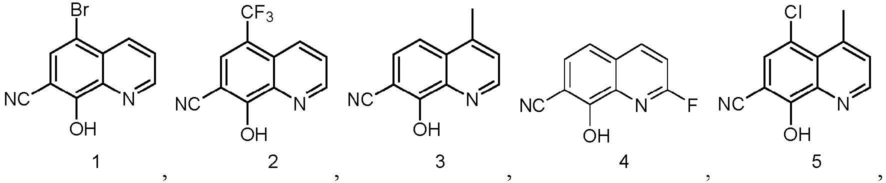

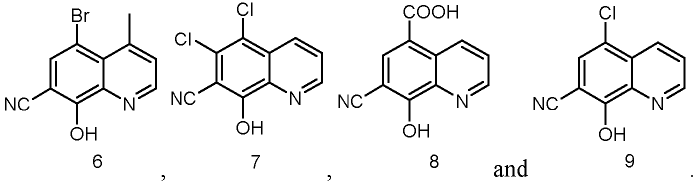

9. A method for preparing the compound of formula (I), the stereoisomer thereof, or the pharmaceutically acceptable salt thereof according to any one of claims 1 to 8, comprising the following steps of:
method I: 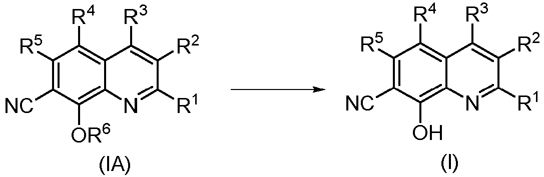
subjecting a compound of formula (IA) to a dealkylation reaction in the presence of lithium chloride to form the compound of formula (I), wherein R⁶ is alkyl, preferably C₁₋₆ alkyl, more preferably methyl;
further, 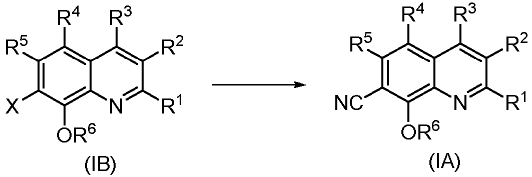
reacting a compound of formula (IA) with zinc cyanide to form the compound of formula (IA), wherein X is a halogen, preferably bromine atom or chlorine atom;
method II: 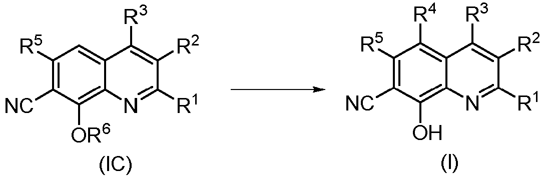
reacting a compound of formula (IA) with a halogenating reagent to form the compound of formula (IA), wherein the halogenating reagent is selected from the group consisting of N-chlorosuccinimide, N-bromosuccinimide, N-iodosuccinimide, pyridinium tribromide, cuprous chloride and cuprous iodide, preferably N-chlorosuccinimide or N-bromosuccinimide, R⁴ is halogen, preferably chlorine atom or bromine atom;
R¹ to R³ and R⁵ are as defined in claim 1.

10. A pharmaceutical composition, comprising a therapeutically effective amount of the compound of formula (I), the stereoisomer thereof, or the pharmaceutically acceptable salt thereof according to any one of claims 1 to 8, and one or more pharmaceutically acceptable excipients.

11. Use of a compound of formula (I'), a stereoisomer thereof, or a pharmaceutically acceptable salt thereof or a pharmaceutical composition comprising the same, in the preparation of a medicament for treating infectious disease or cancer, 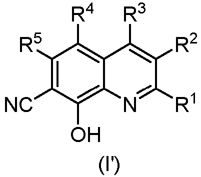 wherein, R¹ to R³ and R⁵ are as defined in claim 1;
R⁴ is selected from the group consisting of hydrogen atom, halogen, alkyl, haloalkyl, carboxy, hydroxy, hydroxyalkyl, alkoxy, haloalkoxy, amino, thiol, alkenyl, alkynyl, cycloalkyl, heterocyclyl, aryl and heteroaryl; preferably, R⁴ is selected from the group consisting of hydrogen atom, halogen, C₁₋₆ alkyl, haloC₁₋₆ alkyl, carboxy and hydroxy;
wherein, the infectious disease is preferably systemic infection, reproductive system infection or urinary tract infection; particularly, the infectious disease is caused by a gram-negative bacterium, a gram-positive bacterium, a virus or a fungus; more particularly, the gram-negative bacterium includes *Escherichia coli, Acinetobacter baumannii* and *Klebsiella pneumoniae,* the *Escherichia coli* is preferably a carbapenem-resistant *Escherichia coli,* and the *Acinetobacter baumannii* is preferably a carbapenem-resistant *Acinetobacter baumannii;* the gram-positive bacterium includes *Staphylococcus aureus,* the *Staphylococcus aureus* is preferably a methicillin-resistant and/or -susceptible *Staphylococcus aureus;* the virus includes human papillomavirus, preferably one or more of *Human Papillomavirus 6 (HPV6), Human Papillomavirus 11 (HPV11), Human Papillomavirus 16 (HPV16)* and *Human Papillomavirus 18 (HPV18),* more preferably *Human papillomavirus 6, Human Papillomavirus 11, Human papillomavirus 16* or *Human Papillomavirus 18;*
wherein, the cancer is preferably bladder cancer or prostate cancer.
